(19) Europäisches Patentamt
European Patent Office
Office européen des brevets

(11) EP 1 697 362 B1

(12) # EUROPEAN PATENT SPECIFICATION

(45) Date of publication and mention of the grant of the patent:
**05.03.2008 Bulletin 2008/10**

(21) Application number: **04806419.0**

(22) Date of filing: **20.12.2004**

(51) Int Cl.:
***C07D 471/16*** *(2006.01)* ***A61K 31/4985*** *(2006.01)*
***A61P 3/04*** *(2006.01)* ***A61P 25/32*** *(2006.01)*
***A61P 25/34*** *(2006.01)* ***A61P 29/00*** *(2006.01)*

(86) International application number:
**PCT/IB2004/004242**

(87) International publication number:
**WO 2005/063749 (14.07.2005 Gazette 2005/28)**

(54) **CRF RECEPTOR ANTAGONISTS AND METHODS RELATING THERETO**

CRF-REZEPTORANTAGONISTEN UND DIESE BETREFFENDE VERFAHREN

ANTAGONISTES DU RECEPTEUR DE CRF ET PROCEDES CORRESPONDANTS

(84) Designated Contracting States:
**AT BE BG CH CY CZ DE DK EE ES FI FR GB GR HU IE IS IT LI LT LU MC NL PL PT RO SE SI SK TR**
Designated Extension States:
**HR LV**

(30) Priority: **22.12.2003 US 532032 P**

(43) Date of publication of application:
**06.09.2006 Bulletin 2006/36**

(73) Proprietors:
- **SB Pharmco Puerto Rico Inc.**
  **00918 San Juan (PR)**
- **NEUROCRINE BIOSCIENCES, INC.**
  **San Diego, CA 92130 (US)**

(72) Inventors:
- **LUO, Zhiyong,**
  **Neurocrine Biosciences Inc**
  **San Diego, California 92130 (US)**
- **TELLEW, John Edward,**
  **Neurocrine Biosciences Inc**
  **San Diego, California 92130 (US)**
- **WILLIAMS, John,**
  **Neurocrine Biosciences Inc**
  **San Diego, California 92130 (US)**

(74) Representative: **Giddings, Peter John**
**GlaxoSmithKline**
**Corporate Intellectual Property (CN9.25.1)**
**980 Great West Road**
**Brentford, Middlesex TW8 9GS (GB)**

(56) References cited:
**WO-A-00/27850** **US-B1- 6 348 466**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 697 362 B1

## Description

TECHNICAL FIELD

This invention relates generally to CRF receptor antagonists and to methods of treating disorders by administration of such antagonists to a mammal in need thereof.

BACKGROUND OF THE INVENTION

The first corticotropin-releasing factor (CRF) was isolated from ovine hypothalami and identified as a 41-amino acid peptide (Vale et al., Science 213:1394-1397, 1981). Subsequently, sequences of human and rat CRF were isolated and determined to be identical, but different from ovine CRF in 7 of the 41 amino acid residues (Rivier et al., Proc. Natl. Acad. Sci. USA 80:4851, 1983; Shibahara et al., EMBO J. 2:775, 1983).

CRF has been found to produce profound alterations in endocrine, nervous and immune system function. CRF is believed to be the major physiological regulator of the basal and stress-release of adrenocorticotropic hormone ("ACTH"), β-endorphin, and other pro-opiomelanocortin ("POMC")-derived peptides from the anterior pituitary (Vale et al., Science 213:1394-1397, 1981). Briefly, CRF is believed to initiate its biological effects by binding to a plasma membrane receptor which has been found to be distributed throughout the brain (DeSouza et al., Science 224:1449-1451, 1984), pituitary (DeSouza et al., Methods Enzymol. 124:560, 1986; Wynn et al., Biochem. Biophys. Res. Comm. 110: 602-608, 1983), adrenals (Udelsman et al., Nature 319:147-150, 1986) and spleen (Webster, E.L., and E.B. DeSouza, Endocrinology 122:609-617, 1988). The CRF receptor is coupled to a GTP-binding protein (Perrin et al., Endocrinology 118:1171-1179, 1986) which mediates CRF-stimulated increase in intracellular production of cAMP (Bilezikjian, L.M., and W.W. Vale, Endocrinology 113:657-662, 1983). The receptor for CRF has now been cloned from rat (Perrin et al., Endo 133(6):3058-3061, 1993), and human brain (Chen et al., PNAS 90(19):8967-8971, 1993; Vita et al., FEBS 335 (1):1-5, 1993). This receptor is a 415 amino acid protein comprising seven membrane spanning domains. A comparison of identity between rat and human sequences shows a high degree of homology (97%) at the amino acid level.

In addition to its role in stimulating the production of ACTH and POMC, CRF is also believed to coordinate many of the endocrine, autonomic, and behavioral responses to stress, and may be involved in the pathophysiology of affective disorders. Moreover, CRF is believed to be a key intermediary in communication between the immune, central nervous, endocrine and cardiovascular systems (Crofford et al., J. Clin. Invest. 90:2555-2564, 1992; Sapolsky et al., Science 238:522-524, 1987; Tilders et al., Regul. Peptides 5:77-84, 1982). Overall, CRF appears to be one of the pivotal central nervous system neurotransmitters and plays a crucial role in integrating the body's overall response to stress.

Administration of CRF directly to the brain elicits behavioral, physiological, and endocrine responses identical to those observed for an animal exposed to a stressful environment. For example, intracerebroventricular injection of CRF results in behavioral activation (Sutton et al., Nature 297:331, 1982), persistent activation of the electroencephalogram (Ehlers et al., Brain Res. 278:332, 1983), stimulation of the sympathoadrenomedullary pathway (Brown et al., Endocrinology 110:928, 1982), an increase of heart rate and blood pressure (Fisher et al., Endocrinology 110:2222, 1982), an increase in oxygen consumption (Brown et al., Life Sciences 30:207, 1982), alteration of gastrointestinal activity (Williams et al., Am. J. Physiol. 253:G582, 1987), suppression of food consumption (Levine et al., Neuropharmacology 22:337, 1983), modification of sexual behavior (Sirinathsinghji et al., Nature 305:232, 1983), and immune function compromise (Irwin et al., Am. J. Physiol. 255:R744, 1988). Furthermore, clinical data suggests that CRF may be hypersecreted in the brain in depression, anxiety-related disorders, and anorexia nervosa. (DeSouza, Ann. Reports in Med. Chem. 25:215-223, 1990). Accordingly, clinical data suggests that CRF receptor antagonists may represent novel antidepressant and/or anxiolytic drugs that may be useful in the treatment of the neuropsychiatric disorders manifesting hypersecretion of CRF.

The first CRF receptor antagonists were peptides (*see, e.g.,* Rivier et al., U.S. Patent No. 4,605,642; Rivier et al., Science 224:889, 1984). While these peptides established that CRF receptor antagonists can attenuate the pharmacological responses to CRF, peptide CRF receptor antagonists suffer from the usual drawbacks of peptide therapeutics including lack of stability and limited oral activity. Some published patent documents include US2002143008, US6348466, W02001083486, and WO2000027850, all of which disclose tetraazaacenaphthylene compounds as CRF antagonists.

Due to the physiological significance of CRF, the development of biologically-active small molecules having significant CRF receptor binding activity and which are capable of antagonizing the CRF receptor remains a desirable goal. Such CRF receptor antagonists would be useful in the treatment of endocrine, psychiatric and neurological conditions or illnesses, including stress-related disorders in general.

While significant strides have been made toward achieving CRF regulation through administration of CRF receptor antagonists, there remains a need in the art for effective small molecule CRF receptor antagonists. There is also a need for pharmaceutical compositions containing such CRF receptor antagonists, to treat, for example, stress-related disorders. The present invention fulfills these needs, and provides other related advantages.

## SUMMARY OF THE INVENTION

In brief, this invention is generally directed to CRF receptor antagonists, and more specifically to CRF receptor antagonists having the following general structure (I):

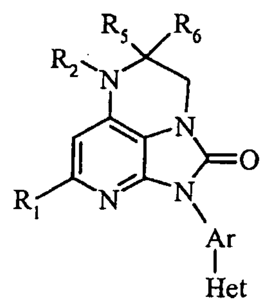

(I)

including stereoisomers, prodrugs and pharmaceutically acceptable salts thereof, wherein $R_1$, $R_2$, $R_5$, Ar, and Het are as defined below.

The CRF receptor antagonists of this invention may have utility over a wide range of therapeutic applications, and may be used to treat a variety of disorders or illnesses, including stress-related disorders. Such methods include administering an effective amount of a CRF receptor antagonist of this invention, preferably in the form of a pharmaceutical composition, to an animal in need thereof. Accordingly, in another embodiment, pharmaceutical compositions are disclosed containing one or more CRF receptor antagonists of this invention in combination with a pharmaceutically acceptable carrier and/or diluent.

These and other aspects of the invention will be apparent upon reference to the following detailed description. To this end, various references are set forth herein which describe in more detail certain procedures, compounds and/or compositions, and are hereby incorporated by reference in their entirety.

## DETAILED DESCRIPTION OF THE INVENTION

The present invention is directed generally to compounds useful as corticotropin-releasing factor (CRF) receptor antagonists.

In a first embodiment, the CRF receptor antagonists of this invention have the following structure (I):

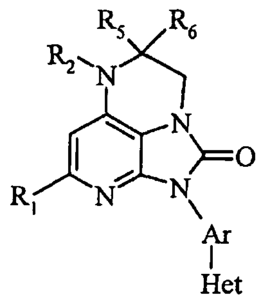

(I)

a compound having the following structure:

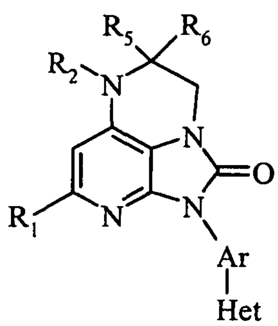

(I)

or a pharmaceutically acceptable salt, ester, solvate, or stereoisomer thereof,
wherein:

$R_1$ and $R_2$ are the same or different and independently are C1-C10 alkyl or substituted C1-C10 alkyl, wherein at least one hydrogen is replaced with a substituent selected among: halogen, hydroxy, C1-C10 alkyl and C1-C10 alkoxy;

Ar is phenyl substituted by one chloro;

Het is pyrazole or dimethylisoxazole;

$R_5$ is hydrogen and $R_6$ is the same or different and is hydrogen, C1-C10 alkyl, or substituted C1-C10 alkyl, wherein at least one hydrogen is replaced with a substituent selected among: halogen, hydroxy, C1-C10 alkyl and C1-C10 alkoxy.

As used herein, the above terms have the following meaning:

"Alkyl" means a straight chain or branched, noncyclic or cyclic, unsaturated or saturated aliphatic hydrocarbon containing from 1 to 10 carbon atoms, while the term "lower alkyl" has the same meaning as alkyl but contains from 1 to 6 carbon atoms. Representative saturated straight chain alkyls include methyl, ethyl, n-propyl, n-butyl, n-pentyl, n-hexyl, and the like; while saturated branched alkyls include isopropyl, sec-butyl, isobutyl, tert-butyl, isopentyl, and the like. Representative saturated cyclic alkyls include cyclopropyl, cyclobutyl, cyclopentyl, cyclohexyl, $-CH_2$-cyclopropyl, $-CH_2$-cyclobutyl, $-CH_2$-cyclopentyl, $-CH_2$-cyclohexyl, and the like; while unsaturated cyclic alkyls include cyclopentenyl and cyclohexenyl, and the like. Cyclic alkyls, also referred to as "homocyclic rings," and include di- and poly-homocyclic rings such as decalin and adamantyl. Unsaturated alkyls contain at least one double or triple bond between adjacent carbon atoms (referred to as an "alkenyl" or "alkynyl", respectively). Representative straight chain and branched alkenyls include ethylenyl, propylenyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, and the like; while representative straight chain and branched alkynyls include acetylenyl, propynyl, 1-butynyl, 2-butynyl, 1-pentynyl, 2-pentynyl, 3-methyl-1 butynyl, and the like.

"Halogen" means fluoro, chloro, bromo and iodo.

"Alkoxy" means an alkyl moiety attached through an oxygen bridge (*i.e.,* -O-alkyl) such as -O-methyl, -O-ethyl, and the like.

Embodiments of this invention presented herein are for purposes of example and not for purposes of limitation. In a first embodiment of the invention, Ar is phenyl optionally substituted by $R_3$ n times where *n* is 1, in the following structure (II), and it is chloro

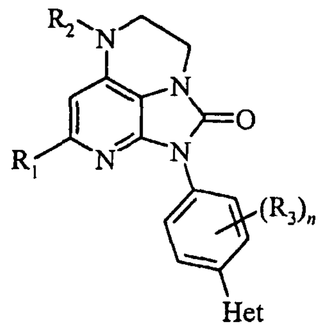

**(II)**

The compounds of the present invention may generally be utilized as the free base. Alternatively, the compounds of this invention may be used in the form of acid addition salts. Acid addition salts of the free base amino compounds of the present invention may be prepared by methods well known in the art, and may be formed from organic and inorganic acids. Suitable organic acids include maleic, fumaric, benzoic, ascorbic, succinic, methanesulfonic, acetic, oxalic, propionic, tartaric, salicylic, citric, gluconic, lactic, mandelic, cinnamic, aspartic, stearic, palmitic, glycolic, glutamic, and benzenesulfonic acids. Suitable inorganic acids include hydrochloric, hydrobromic, sulfuric, phosphoric, and nitric acids. Thus, the term "pharmaceutically acceptable salt" of structure (I) is intended to encompass any and all acceptable salt forms.

In general, the compounds of structure (I) may be made according to the organic synthesis techniques known to those skilled in this field, as well as by the representative methods set forth in the Examples. For example, the synthesis of structure (I) may generally proceed according to the following Reaction Schemes 1 and 2.

Reaction Scheme 1

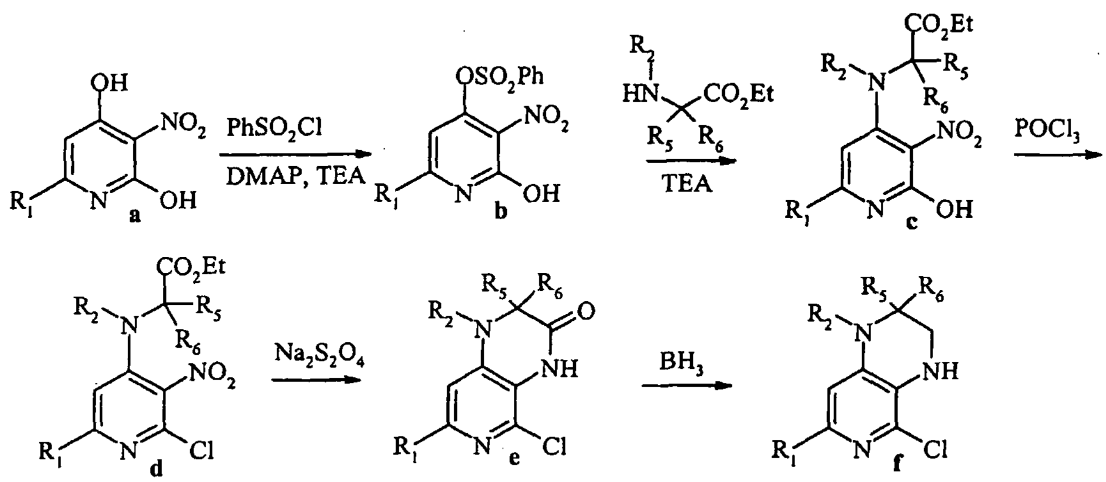


Pyridine a in the presence of DMAP and TEA reacts with benzenesulfonyl chloride to form pyridylphenylsulfonate b. Condensation with alkyl amino acid ester in the presence of base gives aminopyridine c. The 2-chloro derivative d is obtained after reaction of c with phosphorus oxychloride and undergoes ring closure to form the tetrahydropyridopyrazine

e after reaction with sodium hydrosulfite. Compound f is obtained after reduction with, for example, borane.

## Reaction Scheme 2

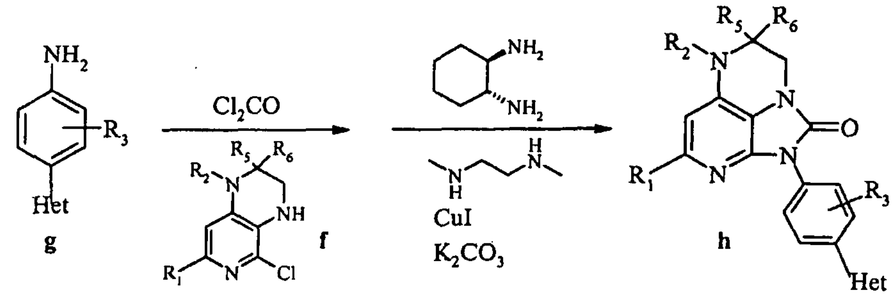


To a solution of aniline g and DIEA in dry DCM is added phosgene. Reaction proceeds overnight, and after evaporation of solvent, tetrahydropyridopyrazine f (Reaction Scheme 1) and DIEA are added in dry DCM. The resulting mixture is stirred and quenched at completion with water. The residue of the dried organic layer is dissolved in 1,4-dioxane, mixed with CuI, $K_2CO_3$, *trans*-1,2-diaminocyclohexane and N,N'-dimethylethylenediamine, and allowed to react overnight in a sealed tube at elevated temperature to obtain compound h after purification. In certain embodiments of the invention, the benzene ring of Cpd g can be replaced by pyridine to afford the N-linked pyridyl analog of Cpd h.

## Reaction Scheme 3

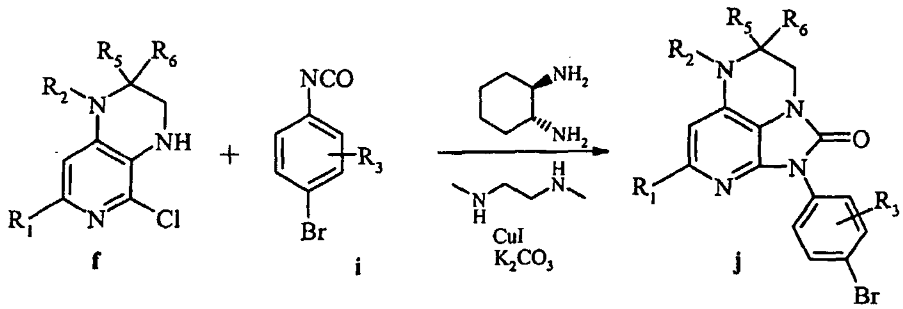


Addition of the phenyl ring to the pyrazolopyridine can be achieved starting with the isocyanate. In Reaction Scheme 3, the (optionally) substituted 4-bromophenylisocyanate i reacts with compound f (Reaction Scheme 1) prior to addition of the N-arylation reagents CuI, $K_2CO_3$ and the diamines. After purification, compound j is obtained.

## Reaction Scheme 4

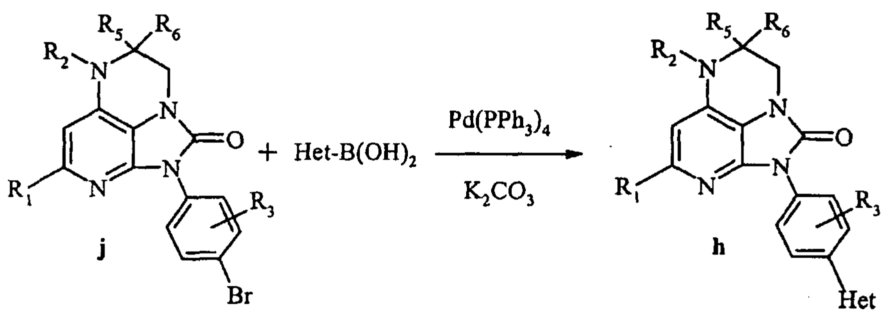


A mixture of compound j with heteroaryl boronic acid, palladium tetrakis(trisphenylphosphine) and $K_2CO_3$ will react with time at elevated temperature to yield compound h.

Reaction Scheme 5

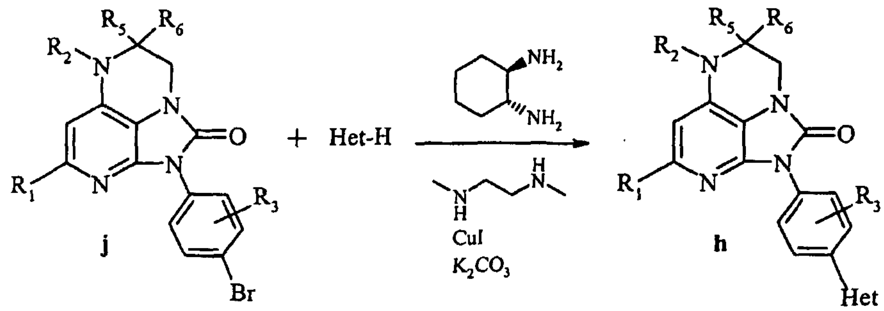


The general procedure of CuI-mediated coupling can be employed in the direct reaction of compound j with a heteroarene, CuI, diamines and $K_2CO_3$ to obtain compound h. In this procedure, the heteroarene must bear an NH group, the nitrogen atom of which becomes coupled to the aryl group of the final product h.

The effectiveness of a compound as a CRF receptor antagonist may be determined by various assay methods. Suitable CRF antagonists of this invention are capable of inhibiting the specific binding of CRF to its receptor and antagonizing activities associated with CRF. A compound of structure (I) may be assessed for activity as a CRF antagonist by one or more generally accepted assays for this purpose, including (but not limited to) the assays disclosed by DeSouza et al. (J. Neuroscience 7:88, 1987) and Battaglia et al. (Synapse 1:572, 1987). As mentioned above, suitable CRF antagonists include compounds which demonstrate CRF receptor affinity. CRF receptor affinity may be determined by binding studies that measure the ability of a compound to inhibit the binding of a radiolabeled CRF (e.g., [$^{125}$I]tyrosine-CFR) to its receptor (e.g., receptors prepared from rat cerebral cortex membranes). The radioligand binding assay described by DeSouza et al. *(supra,* 1987) provides an assay for determining a compound's affinity for the CRF receptor. Such activity is typically calculated from the $IC_{50}$ as the concentration of a compound necessary to displace 50% of the radiolabeled ligand from the receptor, and is reported as a "$K_i$" value calculated by the following equation:

$$K_i = \frac{IC_{50}}{1 + L / K_D}$$

where L = radioligand and $K_D$ = affinity of radioligand for receptor (Cheng and Prusoff, Biochem. Pharmacol. 22:3099, 1973).

In addition to inhibiting CRF receptor binding, a compound's CRF receptor antagonist activity may be established by the ability of the compound to antagonize an activity associated with CRF. For example, CRF is known to stimulate various biochemical processes, including adenylate cyclase activity. Therefore, compounds may be evaluated as CRF antagonists by their ability to antagonize CRF-stimulated adenylate cyclase activity by, for example, measuring cAMP levels. The CRF-stimulated adenylate cyclase activity assay described by Battaglia et al. *(supra,* 1987) provides an assay for determining a compound's ability to antagonize CRF activity. Accordingly, CRF receptor antagonist activity may be determined by assay techniques which generally include an initial binding assay (such as disclosed by DeSouza *(supra,* 1987)) followed by a cAMP screening protocol (such as disclosed by Battaglia *(supra,* 1987)).

With reference to CRF receptor binding affinities, CRF receptor antagonists of this invention have a $K_i$ of less than 10 $\mu$M. In a preferred embodiment of this invention, a CRF receptor antagonist has a $K_i$ of less than 1$\mu$M, and more preferably less than 0.25 $\mu$M (*i.e.,* 250 nM). As set forth in greater detail below, the $K_i$ values may be assayed by the methods set forth in Example 7.

CRF receptor antagonists of the present invention may demonstrate activity at the CRF receptor site, and may be used as therapeutic agents for the treatment of a wide range of disorders or illnesses including endocrine, psychiatric, and neurological disorders or illnesses. More specifically, CRF receptor antagonists of the present invention may be useful in treating physiological conditions or disorders arising from the hypersecretion of CRF. Because CRF is believed to be an important neurotransmitter that activates and coordinates the endocrine, behavioral and automatic responses to stress, CRF receptor antagonists of the present invention may be useful in the treatment of neuropsychiatric disorders. Neuropsychiatric disorders which may be treatable by the CRF receptor antagonists of this invention include affective disorders such as depression; anxiety-related disorders such as generalized anxiety disorder, panic disorder, obsessive-compulsive disorder, abnormal aggression, cardiovascular abnormalities such as unstable angina and reactive hypertension; and feeding disorders such as anorexia nervosa, bulimia, and irritable bowel syndrome. CRF antagonists may

also be useful in treating stress-induced immune suppression associated with various diseases states, as well as stroke. Other uses of the CRF antagonists of this invention may include treatment of inflammatory conditions (such as rheumatoid arthritis, uveitis, asthma, inflammatory bowel disease and G.I. motility), pain, Cushing's disease, infantile spasms, epilepsy and other seizures in both infants and adults, and various substance abuse and withdrawal (including alcoholism).

In another embodiment of the invention, pharmaceutical compositions containing one or more CRF receptor antagonists are disclosed. For the purposes of administration, the compounds of the present invention may be formulated as pharmaceutical compositions. Pharmaceutical compositions of the present invention comprise a CRF receptor antagonist of the present invention (*i.e.,* a compound of structure (I)) and a pharmaceutically acceptable carrier and/or diluent. The CRF receptor antagonist is present in the composition in an amount which is effective to treat a particular disorderthat is, in an amount sufficient to achieve CRF receptor antagonist activity, and preferably with acceptable toxicity to the patient. The pharmaceutical compositions of the present invention may include a CRF receptor antagonist in an amount from 0.1 mg to 250 mg per dosage depending upon the route of administration, and more typically from 1 mg to 60 mg. Appropriate concentrations and dosages can be readily determined by one skilled in the art.

Pharmaceutically acceptable carrier and/or diluents are familiar to those skilled in the art. For compositions formulated as liquid solutions, acceptable carriers and/or diluents include saline and sterile water, and may optionally include antioxidants, buffers, bacteriostats and other common additives. The compositions can also be formulated as pills, capsules, granules, or tablets which contain, in addition to a CRF receptor antagonist, diluents, dispersing and surface active agents, binders, and lubricants. One skilled in this art may further formulate the CRF receptor antagonist in an appropriate manner, and in accordance with accepted practices, such as those disclosed in Remington's Pharmaceutical Sciences, Gennaro, Ed., Mack Publishing Co., Easton, PA 1990.

With regard to stereoisomers, the compounds of structure (I) may have chiral centers and may occur as racemates, racemic mixtures and as individual enantiomers or diastereomers. All such isomeric forms are included within the present invention, including mixtures thereof. Furthermore, some of the crystalline forms of the compounds of structure (I) may exist as polymorphs, which are included in the present invention. In addition, some of the compounds of structure (I) may also form solvates with water or other organic solvents. Such solvates are similarly included within the scope of this invention.

In another embodiment, the present invention provides pharmaceutical compositions useful for treating a variety of disorders or illnesses, including endocrine, psychiatric and neurological disorders or illnesses. Such pharmaceutical compositions include administering of a compound of the present invention to a mammal (e.g., a person) in an amount sufficient to treat the disorder or illness. As used herein, systemic administration includes oral and parenteral methods of administration. For oral administration, suitable pharmaceutical compositions of CRF receptor antagonists include powders, granules, pills, tablets, and capsules as well as liquids, syrups, suspensions, and emulsions. These compositions may also include flavorants, preservatives, suspending, thickening and emulsifying agents, and other pharmaceutically acceptable additives. For parental administration, the compounds of the present invention can be prepared in aqueous injection solutions which may contain, in addition to the CRF receptor antagonist, buffers, antioxidants, bacteriostats, and other additives commonly employed in such solutions. ,

In another embodiment, the present invention permits the diagnostic visualization of specific sites within the body by the use of radioactive or non-radioactive pharmaceutical agents Use of a compound of the present invention may provide a physiological, functional, or biological assessment of a patient or provide disease or pathology detection and assessment. Radioactive pharmaceuticals are employed in scintigraphy, positron emission tomography (PET), computerized tomography (CT), and single photon emission computerized tomography (SPECT.) For such applications, radioisotopes are incorporated of such elements as iodine (I) including $^{123}$I (PET), $^{125}$I (SPECT), and $^{131}$I, technetium (Tc) including $^{99}$Tc (PET), phosphorus (P) including $^{31}$P and $^{32}$P, chromium (Cr) including $^{51}$Cr, carbon (C) including "C, fluorine (F) including $^{18}$F, thallium (TI) including $^{201}$TI, and like emitters of positron and ionizing radiation. Non-radioactive pharmaceuticals are employed in magnetic resonance imaging (MRI), fluoroscopy, and ultrasound. For such applications, isotopes are incorporated of such elements as gadolinium (Gd) including $^{153}$Gd, iron (Fe), barium (Ba), manganese (Mn), and thallium (TI). Such entities are also useful for identifying the presence of particular target sites in a mixture and for labeling molecules in a mixture.

As mentioned above, administration of a compound of the present invention can be used to treat a wide variety of disorders or illnesses. In particular, the compounds of the present invention may be administered to a mammal for the treatment of depression, anxiety disorder, panic disorder, obsessive-compulsive disorder, abnormal aggression, unstable angina, reactive hypertension, anorexia nervosa, bulimia, irritable bowel syndrome, stress-induced immune suppression, stroke, inflammation, pain, Cushing's disease, infantile spasms, epilepsy, and substance abuse or withdrawal.

The following examples are provided for purposes of illustration, not limitation.

EXAMPLES

Preparative HPLC-MS

Platform: Shimadzu HPLC equipped with a Gilson 215 auto-sampler/fraction collector, UV detector and a PE Sciex API150EX mass detector;
HPLC column: BHK ODS-O/B, 5 μ, 30x75 mm
HPLC gradient: 35 mL/minute, 10% acetonitrile in water to 100 % acetonitrile in 7 minutes, maintaining 100 % acetonitrile for 3 minutes, with 0.025% TFA.

Analytical HPLC-MS - Method 1

Platform: Agilent 1100 series: equipped with auto-sampler, UV detector (220 nM and 254 nM) and MS detector (APCI);
HPLC column: Phenomenex Synergi-Max RP, 2.0 x 50 mm column;
HPLC gradient: 1.0 mL/minute, from 5% acetonitrile in water to 95% acetonitrile in water in 13.5 min, maintaining 95% acetonitrile for 2 min, both acetonitrile and water having 0.025% TFA.

Analytical HPLC-MS - Method 2

Platform: Agilent 1100 series: equipped with auto-sampler, UV detector (220 nM and 254 nM), MS detector (APCI) and Berger FCM 1200 $CO_2$ pump module;
HPLC column: Berger Pyridine, PYR 60A, 6μ, 4.6 x 150 mm column;
HPLC gradient: 4.0 mL/minute. 120 bar; from 10 % methanol in supercritical $CO_2$ to 60% methanol in supercritical $CO_2$ in 1.67 minutes, maintaining 60 % for 1 minute. Methanol has 1.5% water. Backpressure regulated at 140 bar.

Abbreviations:

AA: Acetic anhydride
Boc-Phe-CHO: (S)-(tertbutoxycarbonylamino)-3-phenylpropional
BOC: *tert*-butoxycarbonyl
DCM: dichloromethane
DMF: dimethylformamide
DMSO: dimethylsulfoxide
EDC: 1-(3-dimethylaminopropyl)-3-ethylcarbodiimide hydrochloride
FMOC: *N*-(9-fluorenylmethoxycarbonyl)
HOBt: 1-hydroxybenzotriazole hydrate
$NaBH(OAc)_3$: Sodium Triacetoxyborohydride
Pd-C: Palladium (10 %) on Carbon
TFA: Trifluoroacetic acid
THF: Tetrahydrofuran

The CRF receptor antagonists of this invention may be prepared by the methods disclosed in Examples 1 to 6. Example 7 presents a method for determining the receptor binding activity ($K_1$).

EXAMPLE 1

SYNTHESIS OF REAGENT 5-CHLORO-1-(1-ETHYL-PROPYL)-7-METHYL-1,2,3,4-TETRAHYDRO-PYRIDO[3,4-B] PYRAZINE

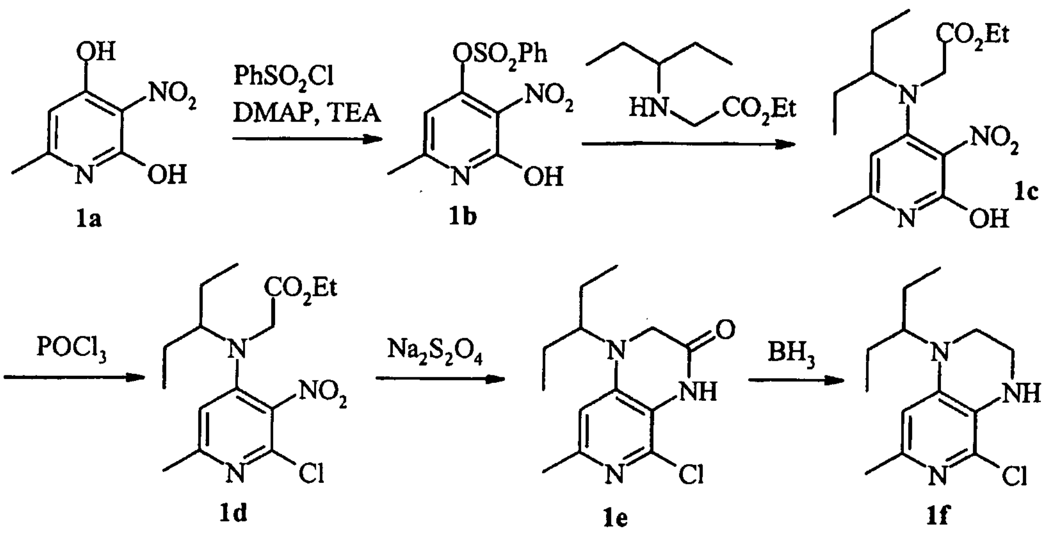


1a 1b 1c 1d 1e 1f

Step 1 A:

To a suspension of 3-nitro-6-methyl-pyridine-2,4-diol **(1a,** 25.5 g) and DMAP (0.92 g) in THF (250 mL) was added TEA (16.7 g) dropwise. The resulting suspension was heated to reflux while benzenesulfonyl chloride (29.2 g) was added dropwise and the mixture was refluxed for an additional 1 hr following completion of the addition. Evaporation of solvent yielded crude **1b.**

Step 1B:

The residue from Step 1A (entire amount) was suspended in MeCN (250 mL.) DMAP (1.0 g) was added followed by dropwise addition of a solution of the amino ester (26.0 g.) in MeCN. The mixture was heated to reflux overnight. After evaporation of solvent, the residue was extracted between EtOAc and aqueous $NaHCO_3$, then the organic layer was dried over sodium sulfate, filtered, and concentrated to yield 1c.

Step 1C:

The crude **1c** was dissolved in MeCN (50 mL) and refluxed with $POCl_3$ (2.0 eq) overnight. The mixture was poured onto ice, then neutralized with sodium carbonate. The mixture was extracted with ethyl acetate. The combined organic extracts were dried over sodium sulfate, filterd, and concentrated. The residue was purified by silica gel chromatography to afford **1d** (10.47 g).

Step 1D:

To a solution of $Na_2S_2O_4$ (26.56 g) and $NaHCO_3$ (12.82 g) in water (100 mL) was added a solution of **1d** (10.45 g) in MeCN (80 mL) dropwise at room temperature. After stirring for 2 hr, the MeCN was evaporated and the residue was extracted with EtOAc. The combined organic extracts were dried over sodium sulfate, filtered, and concentrated to provide crude compound 1e (6.20 g).

Step 1E:

To crude compound **1e** (6.20 g) in dry THF (20 mL) was added borane (1M solution in THF, 3.0 eq) slowly. After stirring at room temperature overnight, methanol was carefully added, then the solvent was evaporated to provide compound **1f** (3.1 g).

EXAMPLE 2

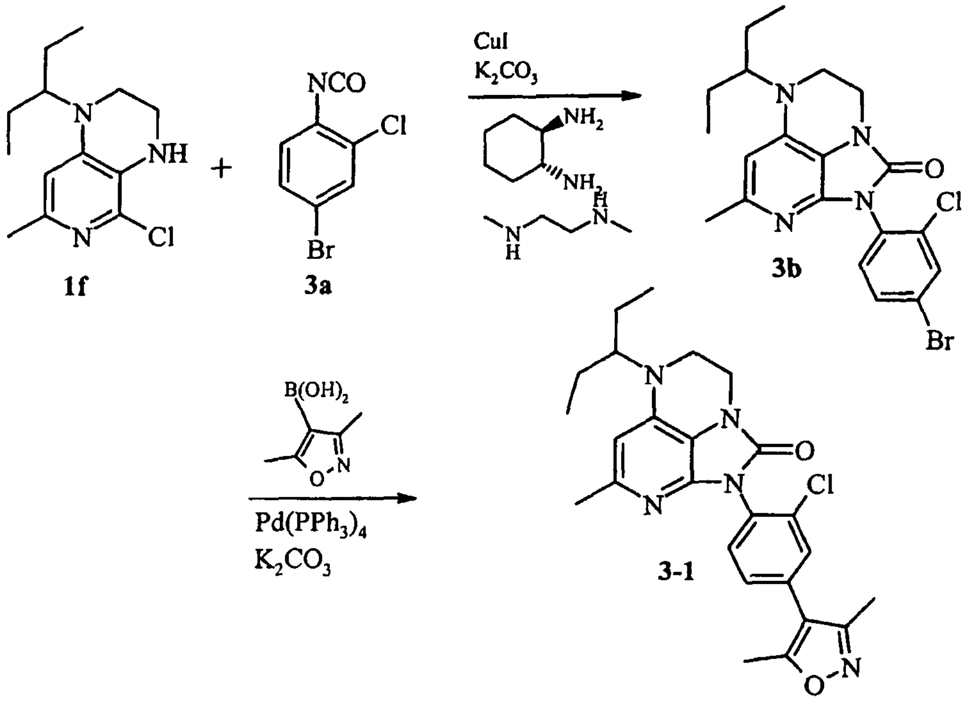


1f 3a 3b 3-1

Step 3A:

A mixture of compound **1f** (253 mg, Example 1) and 2-chloro-4-bromophenyl isocyanate (232 mg) in 1,4-dioxane (2.5 mL) was stirred at room temperature for 5 hr. To the resulting mixture was added CuI (100 mg,) $K_2CO_3$ (414 mg,) *trans*-1,2-diaminocyclohexane (50 uL,) and N,N'-dimethylethylenediamine (50 uL) in turn prior to heating overnight in a sealed tube at 110˚C. The resulting compound 3b (190 mg) was purified by silica gel chromatography.

Step 3B:

A mixture of compound **3b** (25 mg) together with (3,5-dimethyl-isoxazole)-4-boronic acid (0.12 mmol), tetrakis (triphenylphosphine)palladium(0) (0.01 mmol), and $K_2CO_3$ (0.25 mmol) was heated (100 ˚C) in dioxane/water overnight in a sealed tube. Compound **3-1** (8.4 mg) was obtained as a TFA salt after purification via preparative LC-MS. By varying the structure of the heterocycle boronic acid, the compounds in the following table were synthesized:

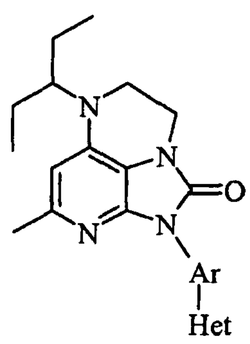

| | Ar | Het | MW | [MH]+ | $t_R$* |
|---|---|---|---|---|---|
| 3-1 | 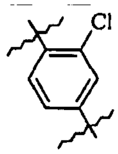 | 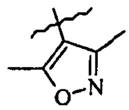 | 465.982 | 610.3 | 1.009 |
| 3-2 | 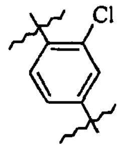 | 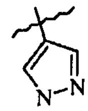 | 436.944 | 437 | 1.422 |
| * All $t_R$ reported for Analytical HPLC Method 2. | | | | | |

EXAMPLE 3

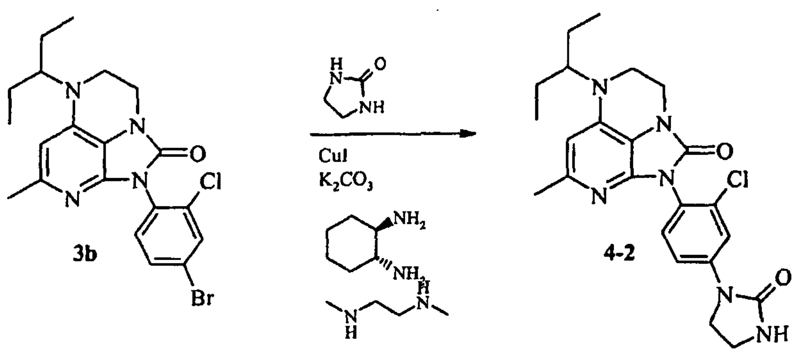


3b

4-2

Step 4A:

Compound **3b** (25 mg) was mixed with imidazolidin-2-one and subjected to the general procedure of CuI-mediate coupling described in Example 3. Compound **4-2** (3.0 mg) was obtained after purification via preparative LC-MS. By varying the heterocycle reactant the following compounds were synthesized.

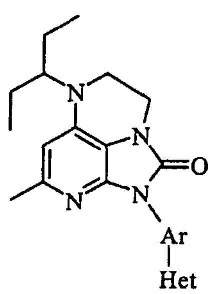

| | Ar | Het | MW | [MH]+ | $t_R$* |
|---|---|---|---|---|---|
| 4-1 | 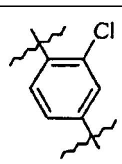 | 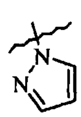 | 436.944 | 437 | 1.168 |

EXAMPLE 4

CRF RECEPTOR BINDING ACTIVITY

The compounds of this invention may be evaluated for binding activity to the CRF receptor by a standard radioligand binding assay as generally described by Grigoriadis et al. (Mol. Pharmacol vol50, pp679-686, 1996) and Hoare et al. (Mol. Pharmacol vol63 pp751-765, 2003.) By utilizing radiolabeled CRF ligands, the assay may be used to evaluate the binding activity of the compounds of the present invention with any CRF receptor subtype.

Briefly, the binding assay involves the displacement of a radiolabeled CRF ligand from the CRF receptor. More specifically, the binding assay is performed in 96-well assay plates using 1-10μg cell membranes from cells stably transfected with human CRF receptors. Each well receives about 0.05 ml assay buffer (e.g., Dulbecco's phosphate buffered saline, 10 mM magnesium chloride, 2mM EGTA) containing compound of interest or a reference ligand (for example, sauvagine, urocortin I or CRF), 0.05 ml of [$^{125}$I] tyrosine - sauvagine (final concentration -150 pM or approximately the $K_D$ as determined by Scatchard analysis) and 0.1 ml of a cell membrane suspension containing the CRF receptor. The mixture is incubated for 2 hours at 22 ˚C followed by separation of the bound and free radioligand by rapid filtration over glass fiber filters. Following three washes, the filters are dried and radioactivity (Auger electrons from $^{125}$I) is counted using a scintillation counter. All radioligand binding data may be analyzed using the non-linear least-squares curve-fitting programs Prism (GraphPad Software Inc) or XLfit (ID Business Solutions Ltd).

EXAMPLE 5

CRF-STIMULATED ADENYLATE CYCLASE ACTIVITY

The compounds of the present invention may also be evaluated by various functional testing. For example, the compounds of the present invention may be screened for CRF-stimulated adenylate cyclase activity. An assay for the determination of CRF-stimulated adenylate cyclase activity may be performed as generally described by Battaglia et al. (*Synapse 1*:572, 1987) with modifications to adapt the assay to whole cell preparations.

More specifically, the standard assay mixture may contain the following in a final volume of 0.1 ml: 2 mM L-glutamine, 20 mM HEPES, and 1 mM IMBX in DMEM buffer. In stimulation studies, whole cells with the transfected CRF receptors are plated in 96-well plates and incubated for 30 min at 37 ˚C with various concentrations of CRF-related and unrelated peptides in order to establish the pharmacological rank-order profile of the particular receptor subtype. Following the incubation, cAMP in the samples is measured using standard commercially available kits, such as cAMP-Screen™ from Applied Biosystems. For the functional assessment of the compounds, cells and a single concentration of CRF or related peptides causing 50% stimulation of cAMP production are incubated along with various concentrations of competing compounds for 30 min at 37˚C, and cAMP determined as described above.

**Claims**

**1.** A compound having the following structure:

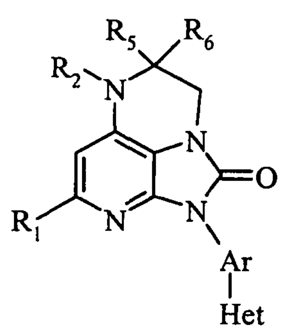

(I)

or a pharmaceutically acceptable salt, ester, solvate, or stereoisomer thereof,
wherein:

$R_1$ and $R_2$ are the same or different and independently are C1-C10 alkyl or substituted C1-C10 alkyl, wherein at least one hydrogen is replaced with a substituent selected among: halogen, hydroxy, C1-C10 alkyl and C1-C10 alkoxy;
Ar is phenyl substituted by one chloro;
Het is pyrazole or dimethylisoxazole;
$R_5$ is hydrogen and $R_6$ is the same or different and is hydrogen, C1-C10 alkyl, or substituted C1-C10 alkyl, wherein at least one hydrogen is replaced with a substituent selected among: halogen, hydroxy, C1-C10 alkyl and C1-C10 alkoxy.

**2.** A composition comprising a compound according to claim 1 and a pharmaceutically acceptable carrier or diluent.

**3.** Use of a compound of formula (I) according to claim 1 in the preparation of a medicament for the treatment treatment of a disorder selected among an affective disorder, an anxiety-related disorder, a feeding disorder, a stress-induced immunosuppressive disorder, an inflammatory disorder or a substance abuse or withdrawal disorder.

**4.** Use of a compound of formula I according to claim 3, wherein the disorder is irritable bowel syndrome.

**5.** Use of a compound of formula I according to claim 3, wherein the disorder is depression.

**6.** Use of a compound of formula I according to claim 3, wherein the disorder is anxiety.

**7.** Use of a compound of formula I according to claim 3, wherein the disorder is obsessive-compulsive disorder.

**8.** Use of a compound of formula I according to claim 3, wherein the disorder is stroke.

**Patentansprüche**

**1.** Verbindung der folgenden Struktur:

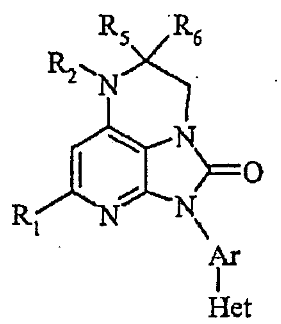

(I)

oder ein pharmazeutisch verträgliches Salz, Ester, Solvat oder Stereoisomer davon, wobei:

$R_1$ und $R_2$ gleich oder verschieden sind und unabhängig voneinander C1-C10-Alkyl oder substituiertes C1-C10-Alkyl sind, wobei mindestens ein Wasserstoff durch einen Substituenten ersetzt ist, ausgewählt aus: Halogen, Hydroxy, C1-C10-Alkyl und C1-C10-Alkoxy;
Ar durch ein Chlor substituiertes Phenyl ist;
Het Pyrazol oder Dimethylisoxazol ist;
$R_5$ Wasserstoff darstellt und $R_6$ gleich oder verschieden ist und Wasserstoff, C1-C10-Alkyl oder substituiertes C1-C10-Alkyl darstellt, wobei mindestens ein Wasserstoff
durch einen Substituenten ersetzt ist, ausgewählt aus: Halogen, Hydroxy, C1-C10-Alkyl und C1-C10-Alkoxy.

**2.** Zusammensetzung umfassend eine Verbindung gemäß Anspruch 1 und einen pharmazeutisch verträglichen Träger

oder Verdünnungsmittel.

**3.** Verwendung einer Verbindung der Formel (I) gemäß Anspruch 1 zur Herstellung eines Medikaments zur Behandlung einer Störung ausgewählt aus einer affektiven Störung, einer Störung in Verbindung mit Angst, einer Fütterungsstörung, einer stressinduzierten immunsuppressiven Störung, einer Entzündungsstörung oder einer Drogenmissbrauchs- oder Entzugsstörung.

**4.** Verwendung einer Verbindung der Formel I gemäß Anspruch 3, wobei die Störung ein Reizdarmsyndrom ist.

**5.** Verwendung einer Verbindung der Formel I gemäß Anspruch 3, wobei die Störung Depression ist.

**6.** Verwendung einer Verbindung der Formel I gemäß Anspruch 3, wobei die Störung Angst ist.

**7.** Verwendung einer Verbindung der Formel I gemäß Anspruch 3, wobei die Störung eine Zwangsstörung ist.

**8.** Verwendung einer Verbindung der Formel I gemäß Anspruch 3, wobei die Störung Schlaganfall ist.

## Revendications

**1.** Composé ayant la structure suivante :

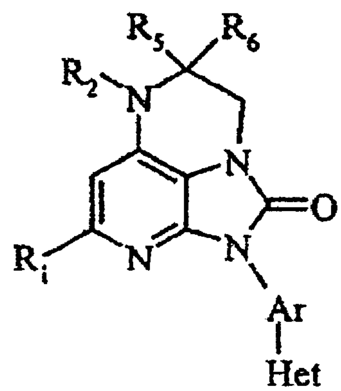

(I)

ou sel, ester, solvate ou stéréoisomère pharmaceutiquement acceptable de celui-ci,
dans lequel :

> $R_1$ et $R_2$ sont identiques ou différents et représentent indépendamment un groupe alkyle en $C_1$ à $C_{10}$ ou un groupe alkyle en $C_1$ à $C_{10}$ substitué, dans lequel au moins un atome d'hydrogène est remplacé par un substituant choisi parmi : un atome d'halogène, un groupe hydroxy, un groupe alkyle en $C_1$ à $C_{10}$ et un groupe alkoxy en $C_1$ à $C_{10}$;
> Ar est un groupe phényle substitué par un groupe chloro ;
> Het est un groupe pyrazole ou diméthylisoxazole ;
> $R_5$ est un atome d'hydrogène et $R_6$ est identique ou différent et représente un atome d'hydrogène, un groupe alkyle en $C_1$ à $C_{10}$, ou un groupe alkyle en $C_1$ à $C_{10}$ substitué, dans lequel au moins un atome d'hydrogène est remplacé par un substituant choisi parmi : un atome d' halogène, un groupe hydroxy, un groupe alkyle en $C_1$ à $C_{10}$ et un groupe alkoxy en $C_1$ à $C_{10}$.

**2.** Composition comprenant un composé selon la revendication 1 et un support ou un diluant pharmaceutiquement acceptable.

**3.** Utilisation d'un composé de formule (I) selon la revendication 1 dans la préparation d'un médicament pour le traitement d'un trouble choisi parmi un trouble affectif, un trouble en rapport avec l'anxiété, un trouble de l'alimentation, un trouble immunosuppresseur induit par un stress, un trouble inflammatoire ou bien un trouble lié à l'abus de substances toxiques ou un trouble de sevrage.

**4.** Utilisation d'un composé de formule I selon la revendication 3, dans laquelle le trouble est le syndrome du côlon irritable.

**5.** Utilisation d'un composé de formule I selon la revendication 3, dans laquelle le trouble est une dépression.

**6.** Utilisation d'un composé de formule I selon la revendication 3, dans laquelle le trouble est l'anxiété.

**7.** Utilisation d'un composé de formule I selon la revendication 3, dans laquelle le trouble est un trouble obsessionnel compulsif.

**8.** Utilisation d'un composé de formule I selon la revendication 3, dans laquelle le trouble est une attaque.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4605642 A, Rivier
- US 2002143008 A
- US 6348466 B
- WO 2001083486 A
- WO 2000027850 A


### Non-patent literature cited in the description


- **VALE et al.** *Science,* 1981, vol. 213, 1394-1397
- **RIVIER et al.** *Proc. Natl. Acad. Sci. USA,* 1983, vol. 80, 4851
- **SHIBAHARA et al.** *EMBO J.,* 1983, vol. 2, 775
- **DESOUZA et al.** *Science,* 1984, vol. 224, 1449-1451
- **DESOUZA et al.** *Methods Enzymol.,* 1986, vol. 124, 560
- **WYNN et al.** *Biochem. Biophys. Res. Comm.,* 1983, vol. 110, 602-608
- **UDELSMAN et al.** *Nature,* 1986, vol. 319, 147-150
- **WEBSTER, E.L. ; E.B. DESOUZA.** *Endocrinology,* 1988, vol. 122, 609-617
- **PERRIN et al.** *Endocrinology,* 1986, vol. 118, 1171-1179
- **BILEZIKJIAN, L.M. ; W.W. VALE.** *Endocrinology,* 1983, vol. 113, 657-662
- **PERRIN et al.** *Endo,* 1993, vol. 133 (6), 3058-3061
- **CHEN et al.** *PNAS,* 1993, vol. 90 (19), 8967-8971
- **VITA et al.** *FEBS,* 1993, vol. 335 (1), 1-5
- **CROFFORD et al.** *J. Clin. Invest.,* 1992, vol. 90, 2555-2564
- **SAPOLSKY et al.** *Science,* 1987, vol. 238, 522-524
- **TILDERS et al.** *Regul. Peptides,* 1982, vol. 5, 77-84
- **SUTTON et al.** *Nature,* 1982, vol. 297, 331
- **EHLERS et al.** *Brain Res.,* 1983, vol. 278, 332
- **BROWN et al.** *Endocrinology,* 1982, vol. 110, 928
- **FISHER et al.** *Endocrinology,* 1982, vol. 110, 2222
- **BROWN et al.** *Life Sciences,* 1982, vol. 30, 207
- **WILLIAMS et al.** *Am. J. Physiol.,* 1987, vol. 253, G582
- **LEVINE et al.** *Neuropharmacology,* 1983, vol. 22, 337
- **SIRINATHSINGHJI et al.** *Nature,* 1983, vol. 305, 232
- **IRWIN et al.** *Am. J. Physiol.,* 1988, vol. 255, R744
- **DESOUZA.** *Ann. Reports in Med. Chem.,* 1990, vol. 25, 215-223
- **RIVIER et al.** *Science,* 1984, vol. 224, 889
- **DESOUZA et al.** *J. Neuroscience,* 1987, vol. 7, 88
- **BATTAGLIA et al.** *Synapse,* 1987, vol. 1, 572
- **CHENG ; PRUSOFF.** *Biochem. Pharmacol.,* 1973, vol. 22, 3099
- Remington's Pharmaceutical Sciences. Mack Publishing Co, 1990
- **GRIGORIADIS et al.** *Mol. Pharmacol,* 1996, vol. 50, 679-686
- **HOARE et al.** *Mol. Pharmacol,* 2003, vol. 63, 751-765